(19) Europäisches Patentamt
European Patent Office
Office européen des brevets

(11) **EP 2 472 259 B1**

(12) **EUROPÄISCHE PATENTSCHRIFT**

(45) Veröffentlichungstag und Bekanntmachung des Hinweises auf die Patenterteilung:
**19.02.2014  Patentblatt 2014/08**

(51) Int Cl.:
*G01N 33/53* (2006.01)    *C07K 7/08* (2006.01)

(21) Anmeldenummer: **12000261.3**

(22) Anmeldetag: **21.06.2007**

(54) **Spezifisch an einen Köder bindende Moleküle**

Specific molecules clinging to a lure

Molécules liées à un hameçon de manière spécifique

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **29.06.2006  DE 102006030028**

(43) Veröffentlichungstag der Anmeldung:
**04.07.2012  Patentblatt 2012/27**

(62) Dokumentnummer(n) der früheren Anmeldung(en) nach Art. 76 EPÜ:
**10013453.5 / 2 306 193**
**07012129.8 / 1 873 524**

(73) Patentinhaber: **Forschungszentrum Jülich GmbH**
**52425 Jülich (DE)**

(72) Erfinder:
- **Willbold, Dieter**
  **52428 Jülich (DE)**
- **Hoffmann, Silke**
  **61250 Usingen (DE)**
- **Wiesehan, Katja**
  **82069 Hohenschäftlarn (DE)**
- **Bujnicki, Tran, Thi Tuyen**
  **12355 Berlin (DE)**
- **Mödder, Susanne**
  **52080 Aachen (DE)**
- **Aladag, Amine**
  **47798 Krefeld (DE)**
- **Glück, Julian**
  **40667 Meerbusch (DE)**

(56) Entgegenhaltungen:
**WO-A-01/79479     US-A1- 2006 115 874**

- **GEIGER A ET AL: "RNA APTAMERS THAT BIND L-ARGININE WITH SUB-MICROMOLAR DISSOCIATION CONSTANTS AND HIGH ENANTIOSELECTIVITY", NUCLEIC ACIDS RESEARCH, OXFORD UNIVERSITY PRESS, SURREY, GB, Bd. 24, Nr. 6, 15. März 1996 (1996-03-15), Seiten 1029-1036, XP002037703, ISSN: 0305-1048**
- **TRAN TUYEN ET AL: "Insights into human Lck SH3 domain binding specificity: Different binding modes of artificial and native ligands", BIOCHEMISTRY, AMERICAN CHEMICAL SOCIETY, EASTON, PA.; US, Bd. 44, Nr. 45, 1. November 2005 (2005-11-01), Seiten 15042-15052, XP002430256, ISSN: 0006-2960**
- **SILKE HOFFMANN ET AL: "Competitively selected protein ligands pay their increase in specificity by a decrease in affinity", MOLECULAR BIOSYSTEMS, Bd. 6, Nr. 1, 1. Januar 2010 (2010-01-01), Seite 126, XP55027668, ISSN: 1742-206X, DOI: 10.1039/b910945e**

**Beschreibung**

Die Erfindung betrifft spezifisch an einen Köder bindende Moleküle.

In vitro Selektionsmethoden, wie z. B. Phagedisplay ermöglichen das Auffinden artifizieller Liganden wie Peptidliganden als Beispiel für Biomoleküle zu einem bestimmten Zielmolekül, das in der Regel als Köder bezeichnet wird.

Aus der Sequenz der erhaltenen Liganden lassen sich wertvolle Rückschlüsse ziehen:

o die Möglichkeit durch Sequenzähnlichkeiten bisher unbekannte natürliche Liganden zuordnen zu können;
o den Einblick in die Bindungsspezifität des Zielmoleküls (welche Voraussetzungen muss ein Ligand erfüllen, damit er an das Zielmolekül binden kann), ergibt sich aus der Consensus-Sequenz der verschiedenen Liganden bzw. aus einzelnen Ligandensequenzen.

Als nach dem Stand der Technik bekannte Selektionsmethode kann beispielhaft die Veröffentlichung von Parmley SF, Smith GP. Antibody-selectable filamentous fd phage vectors: affinity purification of target genes. Gene. 1988 Dec 20; 73(2):305-18, genannt werden. Konventionelle Selektionsmethoden führen per se nicht zu Liganden, die ausschließlich an das eingesetzte Zielmolekül binden. Dieses Problem wird besonders dann bedeutsam, wenn es zum eingesetzten Zielmolekül noch weitere natürlich vorkommende Moleküle mit ähnlichen Struktur- und Funktionseigenschaften gibt.

Dies führt dazu, dass zwar häufig sehr affine Liganden per in vitro Selektion für ein bestimmtes Zielmolekül aufgefunden werden können. Diese haben jedoch nur einen eingeschränkten praktischen Nutzen, weil sie unter natürlichen Bedingungen auch noch eine Vielzahl weiterer Bindungen außer zu dem in der Selektion eingesetzten Zielmolekül eingehen können. Diese "unspezifischen" Wechselwirkungen sind jedoch häufig nicht gewünscht, da sie beispielsweise unerwünschte Modulation von Signalwegen betreffen, denen das eigentliche Zielmolekül nicht angehört.

Es ist daher die Aufgabe der Erfindung, ein Verfahren zum Auffinden von Liganden bzw. Molekülen, insbesondere Biomolekülen, die eine spezifische Bindung zu einem bestimmten Köder aufweisen und hauptsächlich an den eingesetzten Köder nicht jedoch an mit ihm verwandte Moleküle mit ähnlichen Struktur- und Funktionseigenschaften binden, zur Verfügung zu stellen. Im Sinne der Erfindung sind diese spezifisch an einen Köder bindenden Liganden Moleküle, die gegenüber einem bestimmten Köder eine erhöhte Selektivität aufweisen, das heißt, an einen bestimmten Köder stärker binden als zum Köder ähnliche Ködermoleküle. Bevorzugt binden die Liganden ausschließlich an einen bestimmten Köder. Bei Anwesenheit konkurrierender oder kompetitierender Moleküle soll der durch das Verfahren selektierte Ligand bevorzugt an das Zielmolekül binden. Bevorzugt bedeutet im Sinne der Erfindung, dass der Quotient aus der relativen Bindungsaktivität von Kompetitor/Ligand und der relativen Bindungsaktivität von Köder/Ligand immer kleiner als 1 ist, bzw. der Quotient aus der Dissotiationskonstanten aus Kompetitor/Ligand und der Dissotiationskonstanten aus Köder/Ligand größer als 1 ist. Die Spezifität oder Selektivität ist dabei nicht mit der Affinität zu verwechseln, die sich aus der Bindungsstärke von Liganden an einen Köder ergibt. Konkurrieren verschiedene Liganden um den gleichen Köder, so kann der weniger spezifische oder selektive die größere Bindungsaffinität besitzen. Erfindungsgemäß soll derjenige Ligand ermittelt werden, welcher besonders spezifisch an einen Köder bindet. Die Biomoleküle sollen als therapeutische Mittel und Heilmittel geeignet sein und insbesondere die Pathogenität viraler Erkrankungen, wie HIV-1 und Hepatitis C senken.

Ausgehend vom Oberbegriff des Anspruchs 1 wird die Aufgabe erfindungsgemäß gelöst mit den im kennzeichnenden Teil des Anspruchs 1 angegebenen Merkmalen.

Mit dem erfindungsgemäßen Verfahren ist es nunmehr möglich, Biomoleküle bereitzustellen, die biologische Wirkung entfalten und beispielsweise die Pathogenität von Viren wie beispielsweise HIV-1 (Nef-Protein) und Hepatitis C (NS5A-Protein) senken. Die aufgefundenen Biomoleküle binden spezifisch an den vorgegebenen Köder.

Vorteilhafte Weiterbildungen der Erfindung sind in den Unteransprüchen angegeben und ergeben sich aus der Beschreibung sowie den Figuren.

Die Figuren zeigen einen schematischen Verlauf des erfindungsgemäßen Verfahrens sowie Ergebnisse aus den Beispielen.

Es zeigt:

Fig. 1:     Den wesentlichen Schritt des erfindungsgemäßen Verfahrens in schematischer Form.

Fig.2a)-f):     Ergebnisse des Spezifitätsvergleichs.

Im Folgenden soll die Erfindung in ihrer allgemeinen Form erläutert werden:

Das erfindungsgemäße Verfahren umfasst folgende Schritte:

a) Immobilisierung eines als Köder fungierenden Moleküls an einer Oberfläche bzw. Bereitstellen eines immobilisierten Köders.

b) In Kontakt bringen des immobilisierten Köders mit einer Lösung, die eine Bibliothek von Molekülen enthält.

c) In Kontakt bringen der mit Molekülen besetzten immobilisierten Köder mit Kompetitoren.

d) Vermehrung der nach dem Spezifitätswaschschritt c) weiterhin an den Köder gebundenen Moleküle.

e) Wiederholung der Schritte b) bis d), wobei die Konzentration der Kompetitoren pro Selektionsrunde vorzugsweise erhöht wird.

f) Identifizierung der Struktur der nach der letzten Selektionsrunde verbleibenden Moleküle.

Bei dem Köder nach Punkt a) handelt es sich um eine Verbindung, an die das zu selektierende Biomolekül gebunden werden soll. Er wird nach dem Stand der Technik bekannten Methoden an eine Oberfläche fixiert. Beispielhaft aber nicht beschränkend können als Köder Proteine, Peptide, RNA oder DNA-Moleküle genannt werden. Als mögliche Oberflächen können beispielhaft Mikrotiterplatten, Magnetpartikel, Agarose- oder Sepharosekügelchen verwendet werden.

Im zweiten Schritt b) wird der immobilisierte Köder mit einer randomisierten Bibliothek von Molekülen - speziell Biomolekülen - in Kontakt gebracht. Diese Biomoleküle konkurrieren um die Bindung an den Köder. Bei der randomisierten Bibliothek handelt es sich um eine Mischung von sehr vielen, beispielsweise $10^{12}$, aber auch $10^4$ oder nur 100 verschiedenen Molekülen in einer Mischung. Eine solche Bibliothek kann beispielsweise jeweils aus Peptiden, Proteinen, DNA, RNA oder m-RNA bestehen, welche an bestimmten Vehikeln gebunden vorliegen, und die an Köder anbinden können. Als Vehikel kommen beispielsweise Phagen, Polysomen oder Bakterienoberflächen in Betracht. Die Bibliothek kann aus artifiziellen oder aus der Natur isolierten Bestandteilen oder aus einer Mischung von beidem bestehen. Unter artifiziell im Sinne der Erfindung sind beispielsweise aus der Oligonukleotidsynthese hergestellte Verbindungen zu verstehen.

Erfindungsgemäß wird der mit Biomolekülen besetzte immobilisierte Köder in Schritt c) mit Kompetitoren in Kontakt gebracht. Hierzu wird in Schritt c) ein Spezifitätswaschschritt durchgeführt, bei dem der Lösung Kompetitoren zugefügt werden, bzw. die immobile Phase mit einer Lösung, die Kompetitoren enthält, gespült wird oder die Lösung mit der Bibliothek von Biomolekülen vorzugsweise wiederholt durch eine Lösung ausgetauscht wird, die Kompetitoren enthält. Bei den Kompetitoren handelt es sich um köderähnliche Moleküle, deren Bindestellen Ähnlichkeiten mit denen des immobilisierten Köders aufweisen. Die in Lösung vorliegenden Kompetitoren konkurrieren im Waschschritt um die schon an den immobilisierten Köder gebundenen Bibliotheksmoleküle. Somit werden diejenigen Bibliotheksmoleküle, welche dem eigentlich "besten" Bibliotheksmitglied für den immobilisierten Köder ähnlich sind, jedoch besser an einen der freien Kompetitoren binden, dem immobilisierten Köder wieder entzogen. Die Schnelligkeit der Ablösungsreaktion der bindenden Bibliotheksmoleküle wird dabei hauptsächlich durch die unterschiedlichen Dissoziationskonstanten ($k_{off}$-Werte) der einzelnen Moleküle bestimmt. Solche mit einem kleinen $k_{off}$-Wert bleiben statistisch gesehen am längsten am immobilisierten Köder gebunden und haben damit eine geringere statistische Wahrscheinlichkeit, eine Bindung mit den angebotenen Kompetitormolekülen eingehen zu können. Solche Bibliotheksmoleküle zeigen letztendlich eine spezifische bzw. selektive Bindung an den Köder. Beispielhaft aber nicht beschränkend können Proteine, Peptide, DNA oder RNA als Kompetitoren genannt werden. Die die Kompetitoren enthaltende Flüssigkeit ist vorzugsweise wässrig und kann einen pH-Puffer enthalten.

Weitere fakultative Komponenten der Lösung für den Spezifitätswaschschritt sind Salze, Detergentien oder Reduktionsmittel.

Bei der nachfolgenden Vermehrung der noch am Köder verbliebenen Bibliotheksmoleküle nach dem Spezifitätswaschschritt nach Schritt d) werden die gebundenen Biomoleküle vom Köder getrennt und nach bekannten Methoden vermehrt. Hierzu können beispielsweise nach den Schritten a) bis c) gewonnene Phagenpartikel in Zellen eingebracht und vermehrt werden. Die Trennung kann beispielsweise durch Änderung des pH-Wertes, Erhitzen oder Änderung, insbesondere Erhöhung der Salzkonzentration erfolgen.

Bei Schritt e) ist die Konzentration der selektierten Biomoleküle in der Lösung, die dem Köder nach Schritt a) zugeführt wird, erhöht.
Vorzugsweise werden 3 bis 6 Selektionsrunden, die die Schritte a) bis e) enthalten, durchgeführt. Es können aber auch 1- >10 oder 1- >20 Wiederholungen durchgeführt werden. Die dabei vorzugsweise vorgenommene Erhöhung der Kompetitorenkonzentration in Schritt c) bei zunehmender Zyklenzahl führt zu einer verbesserten Selektion. Die Konzentration der Kompetitoren kann anfangs beispielsweise bei 1 nmol/l liegen. Die Konzentrationsänderung der Kompetitoren kann in Schritten von Verdopplung bis Verzehnfachung oder mehr erfolgen. Typische Endkonzentrationen liegen bei 1 $\mu$mol/l.

Die Identifizierung des nach mehreren Zyklen der Schritte a) bis e) erhaltenen Biomoleküls kann durch bekannte Methoden erfolgen.

Erfindungswesentlich ist bei dem erfindungsgemäßen Verfahren die Verwendung von Kompetitoren.

Das erfindungsgemäße Verfahren kann grundsätzlich in alle bekannten Selektionsverfahren, insbesondere in vitro Selektionsprotokolle integriert werden.

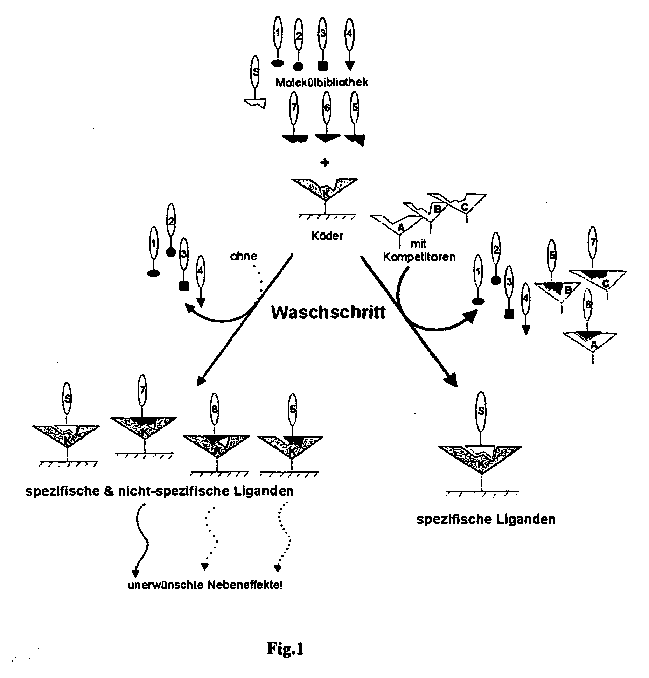

In Figur 1 sind Schritte des erfindungsgemäßen Verfahrens dargestellt.

Sie zeigt die Vorzüge eines Spezifitätswaschschrittes.

Ein beliebiger Köder (K) wird mit einer Molekülbibliothek vermischt. Dabei unterscheiden sich die Bindungseigenschaften der einzelnen Varianten der Molekülbibliothek zum Köder. Es gibt Varianten, die keine Bindung mit dem Köder eingehen können (1, 2, 3, 4). Die Bibliothek enthält weiterhin Varianten (7, 8, 9), die an das Ködermolekül und auch an weitere zum Köder ähnliche Ziele (A, B, C) binden können. Nur wenige Varianten der Bibliothek können an den Köder, jedoch nicht an zu diesem ähnliche Ziele binden. Diese Varianten zeigen spezifische Bindungseigenschaften (S).

In herkömmlichen Selektionsverfahren wird der Standardwaschschritt (links) durchgeführt. Hier bleiben am Ende der Selektion spezifische und unspezifische Varianten am Ködermolekül hängen. Die unspezifischen Varianten führen zu unerwünschten Nebeneffekten. Durch Einführen des Spezifitätswaschschrittes (rechts) kann dies verhindert werden. Hier werden zum Köder ähnliche Ziele als Kompetitoren eingesetzt und somit nichtspezifische Varianten weggewaschen. Übrig bleiben spezifische Liganden, die den Köder, jedoch vorzugsweise keine anderen Ziele binden. Spezifische Liganden können beispielsweise bei der Anwendung in der Medizin ohne Nebeneffekte eingesetzt werden.

Die mit dem erfindungsgemäßen Verfahren erhaltenen Biomoleküle sind ebenfalls Bestandteil der Erfindung:

So sind insbesondere Peptidliganden mit spezifischen Bindungseigenschaften für SH3-Domänen von Src-Tyrosinkinasen, insbesondere für Lck-, Fyn- und Lyn-SH3 Molekülen Bestandteil der Erfindung.

Beispielhaft aber nicht beschränkend können als Biomoleküle die in den Sequenzprotokollen angeführten Proteine angeführt werden, die artifiziellen Ursprungs sind.

Es zeigt:

Seq. Nr.1 ein Protein
Seq. Nr.2 ein Protein
Seq. Nr.3 ein Protein
Seq. Nr.4 ein Protein
Seq. Nr.5 ein Protein

Die erfindungsgemäßen Peptide, die in den Sequenzprotokollen als Proteine klassifiziert sind, können nach bekannten Verfahren hergestellt werden.

So kann zur Herstellung ein chemisch-synthetisches Verfahren zum Beispiel Festphasen-Synthesen oder eine Synthese in flüssigem Medium herangezogen werden. Bei der Festphasensynthese werden die Aminosäuren in der Abfolge der Sequenz entsprechend den Sequenzprotokollen miteinander verbunden. Die Festphasenpeptidsynthese besteht aus drei wesentlichen Schritten.

1) Aufbau der Aminosäurekette zum Peptid,
2) Spaltung des synthetisierten Peptids vom Harz und
3) Reinigung und Charakterisierung.

Für die Synthese der Aminosäurekette sind unterschiedliche Kopplungsmethoden bekannt, wie Sie beispielsweise in "Beyer Walter" 22. Auflage ISBN 3-7776-0485-2 Seite 829-834 beschrieben werden.

Die Peptide können auch durch Expression der für es kodierende Nucleotidsequenzen, zum Beispiel in Chromosomen, Plasmiden oder anderen Informationsträgern, nackte DNA oder RNA in Organismen, in Zellen oder zellfreien Systemen, hergestellt werden.

Gegenstand der Erfindung sind daher auch die Nucleinsäuren, welche für das Peptid gemäß der Aminosäuresequenz gemäß den Sequenzprotokollen 1-5 kodieren, einschließlich aller Allelvariationen.

Der erfindungsgemäße Wirkstoff und die pharmazeutische Zusammensetzung können als flüssige, halbfeste oder feste Arzneiformen und in Form von z. B. Injektionslösungen, Tropfen, Säften, Sirupen, Spray, Suspensionen, Granulaten, Tabletten, Pellets, transdermalen therapeutischen Systemen, Kapseln, Pflastern, Zäpfchen, Salben, Cremes, Lotionen, Gelen, Emulsionen oder Aerosolen vorliegen und verabreicht werden und enthalten die erfindungsgemäßen Peptide in einer physiologisch verträglichen Form und in Abhängigkeit des Applikationsweges pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsmittel können beispielsweise sein: Wasser, Ethanol, 2-Propanol, Glycerin, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokosnussöl, Erdnussöl, Sojabohnenöl, Lecithin, Natriumlactat, Polyoxyethylen- und - propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumoxid, Titanoxid, Titandioxid, Magnesiumsulfat, Zinksulfat, Calciumsulfat, Pottasche, Calciumphosphat, Dicalciumphosphat, Kaliumbromid, Kaliumjodid, Talkum, Kaolin, Pectin, Crospovidon, Agar und Bentonit.

Die Auswahl der Hilfsstoffe sowie der einzusetzenden Mengen derselben hängt davon ab, ob das Medikament oral, subkutan, parenteral, intravenös, pulmonal, intraperitoneal, transdermal, intramuskulär, nasal, buccal, rectal oder auf andere geeignete Weise appliziert werden soll. Für die orale Applikation eignen sich u. a. Zubereitungen in Form von Tabletten, Dragees, Kapseln, Granulaten, Tropfen, Säften und Sirupen; für die parenterale, topische und inhalative Applikation Lösungen, Suspensionen, leicht rekonstruierbare Pulver zur Inhalation sowie Sprays. In geeigneten perkutanen Applikationsformen kann der Wirkstoff in einem Depot in gelöster Form oder in einem Pflaster, gegebenenfalls unter Zusatz von die Hautpenetration fördernden Mitteln vorliegen. Rektal, transmucosal, parenteral, oral oder percutan anwendbare Zubereitungsformen können die erfindungsgemäßen Peptide verzögert freisetzen.

In flüssiger Form können die erfindungsgemäßen Peptide beispielsweise intravenös, oral, als Nasenspray, subcutan, intramuskulär, inhalativ oder in oder neben das Rückenmark appliziert werden. Weiterhin können die erfindungsgemäßen Wirkstoffe mittels Salben oder Cremes aufgebracht werden.

Die erfindungsgemäßen Peptide können am Wirkort oder an anderen Orten des Organismus entstehen, nachdem Nucleinsäuren (DNA und RNA oder eine Kombination davon), die für die erfindungsgemäßen Peptide kodieren, in den Organismus eingebracht werden. Dies kann durch virale Vektoren, nackte Nucleinsäure (DNA,RNA), Plasmide, künstliche Viruspartikel, Liposomen erreicht werden, die intravenös, intranasal, oral, rectal, subcutan, intramuskulär, in oder an das Rückenmark in den Körper eingebracht werden.

Vorteile der Erfindung:

I. Lck-SH3-Liganden als Biomoleküle Seq. Nr: 1 und 2:

1. Spezifische Lck-SH3-Liganden stellen wertvolles Werkzeug für die medizinische Grundlagenforschung dar, da mit ihnen die Aufklärung von Signalwegen möglich ist, an welchen die Lck beteiligt ist.

2. Lck wird hauptsächlich von T-Zellen exprimiert. Dort ist sie Bestandteil des T-Zell-Rezeptorkomplexes. Die erste nachweisbare Veränderung innerhalb der T-Zelle nach Antigenbindung ist die Phosphorylierung von Tyrosinresten, wodurch die Signalkaskade ins Innere der Zelle gestartet wird. Wichtig ist hierbei die Assoziation der Lck-Kinase mit bestimmten Korezeptoren (CD4/CD8) und letztendlich die Aktivierung anderer Kinasen und Adaptermoleküle via Phosphorylierung durch Lck. Bei krankhaft verringerter Lck-Aktivität werden Signale von außen nicht mehr richtig in den Zellkern weitergeleitet und somit erfolgt keine Aktivierung der T-Zellen, was im Verlust der Immunantwort und einem stark verringertem Immunschutz endet (Straus DB, Weiss A. Genetic evidence for the involvement of the lck tyrosine kinase in signal transduction through the T cell antigen receptor.Cell. 1992 Aug 21;70(4):585-93. Erhöhte Lck-Aktivität, wie sie z.B. bei bestimmten Mutationen des lck-Gens bei T-Zell Leukämien und Lymphomen sowie bei T-Zell vermittelten Autoimmunkrankheiten auftreten (Lawrence DS, Niu J. Protein kinase inhibitors: the tyrosine-specific protein kinases.Pharmacol Ther. 1998 Feb;77(2):81-114), haben ebenfalls negative Auswirkungen auf den Organismus. Wegen der regulatorischen Funktion der Lck-SH3-Domäne ist eine begleitende Therapie mit spezifischen Lck-SH3-Liganden bei oben genannten Krankheitsbildern möglich. Die mit dem erfindungsgemäßen Verfahren gefundenen Lch-SH3-Liganden sind in der Lage, die Bindung von physiologischen Cofaktoren und damit die Lck-Aktivität spezifisch zu hemmen. Sie sind daher als Immunsupressiva geeignet, wie sie bei der Behandlung von Transplantationspatienten eingesetzt werden.

3. Im Speziellen kann durch den Einsatz spezifischer Lck-SH3 Liganden die Pathogenität solcher viraler Erreger geschwächt werden, die direkt Effektormoleküle, welche an die SH3-Domäne von Lck binden, besitzen. Hierzu gehören z.B. die menschlichen Krankheitserreger HIV-1 (Nef-Protein) und Hepatitis C (NS5A Protein).

II-Fyn-SH3-Liganden als Biomoleküle Seq. Nr. 3 und 4:

1. Spezifische Fyn-SH3-Liganden stellen wertvolles Werkzeug für die medizinische Grundlagenforschung dar, da mit ihnen die Aufklärung von Signalwegen möglich ist an welchen die Fyn beteiligt ist.

2. Die Phosphotyrosinkinase Fyn wird in einer Vielzahl von Zelltypen exprimiert. So findet sie sich unter anderem in Monozyten, T-/B-Zellen und NK-Zellen. Die biologischen Funktionen sind relativ divers. So spielt Fyn eine Rolle in der T-Zellrezeptor Signaltransduktion, in der Regulation von Gehirnfunktionen, aber auch in adhäsionsvermittelter Signalweiterleitung. Als ein Ergebnis alternativen Splicings, wird Fyn in zwei unterschiedlichen Isoformen exprimiert. In T-Zellen liegt das sogenannte Fyn(T) vor, wobei in Gehirnzellen und anderen Gewebetypen die Fyn(B) Isoform vorzufinden ist. Beide Isoformen unterscheiden sich in annähernd 50 Aminosäuren, die im Bereich des SH2-Kinase-Linkers liegen (Resh MD. Fyn, a Src family tyrosine kinase. Int J Biochem Cell Biol. 1998 Nov;30(11):1159-62).

Zu hohe oder zu niedrige Fyn-Aktivität wurde in mehreren Krebsarten nachgewiesen: z.B. Melanomen (Huang J, et al., Cooperative roles of Fyn and cortactin in cell migration of metastatic murine melanoma. J Biol Chem. 2003 Nov 28; 278 (48):48367-76) oder Neoblastomen (Berwanger B, et al., Loss of a FYN-regulated differentiation and growth arrest pathway in advanced stage neuroblastoma. Cancer Cell. 2002 Nov;2(5):377-86). Wegen der regulatorischen Funktion der Fyn-SH3-Domäne auf die Fyn-Kinaseaktivität ist eine begleitende Therapie mit spezifischen Fyn-SH3-Liganden bei allen Krebsformen, für die eine Beteiligung von Fyn nachgewiesen werden kann, möglich.

III Lyn-SH3-Liganden als Biomoleküle Seq. Nr.5:

1. Spezifische Lyn-SH3-Liganden stellen ein wertvolles Werkzeug für die medizinische Grundlagenforschung dar, da mit ihnen die Aufklärung von Signalwegen möglich ist, an welchen die Lyn beteiligt ist.

2. Lyn wird ebenfalls wie Hck vorwiegend in hämatopoetischen Zelllinien exprimiert und besitzt die Eigenschaft, Signalwege durch Phosphorylierungen von Rezeptoren, Enzymen und Adaptorproteinen negativ zu beeinflussen. Durch die Assoziation mit Oberflächenrezeptoren hämatopoetischer Zellen, wie zum Beispiel dem B-Zell Antigenrezeptor (BCR), dem LPS-Rezeptor und verschiedenen Cytokinrezeptoren, spielt Lyn eine Schlüsselrolle in mehreren Signalwegen der B-Zell-Aktivierung. (Xu Y, et al., Lyn tyrosine kinase: accentuating the positive and the negative. Immunity. 2005 Jan;22(1):9-18).

Zu hohe oder zu niedrige Lyn-Aktivität wurde in mehreren Krebsarten nachgewiesen, z. B. Glioblastomen (Stettner MR, et. al., Lyn kinase activity is the predominant cellular SRC kinase activity in glioblastoma tumor cells. Cancer Res. 2005 Jul 1;65(13):5535-43) und Leukemien (Contri A, et al., Chronic lymphocytic leukemia B cellscontain anomalous Lyn tyrosine kinase, a putative contribution to defective apoptosis. J Clin Invest. 2005 Feb; 115(2):369-78). Wegen der regulatorischen Funktion der Lyn-SH3-Domäne auf die Lyn-Kinaseaktivität ist eine begleitende Therapie mit spezifischen Lyn-SH3-Liganden bei allen Krebsformen, für die eine Beteiligung von Lyn nachgewiesen werden kann, möglich.

Der eingeführte Spezifitätswaschschritt kann auch in anderen gängigen Screeningprozeduren, die alle auf dem Prinzip der Affinitätschromatographie an ein beliebiges Biopolymer und der anschliessenden Amplifikation bindender Moleküle basieren, eingesetzt werden. Die Natur des eingesetzten Köders (Protein, DNA, RNA, chemische Verbindungen) kann dabei variieren. Voraussetzung ist lediglich, dass bekannte Moleküle, die ähnlich zum eingesetzten Köder sind, als Kompetitoren in einem zusätzlichen Waschschritt integriert werden.

Neben Phage-Display wären dies z. B. Spiegelbindphagendisplay (Schumacher TN, Mayr LM, Minor DL Jr, Milhollen MA, Burgess MW, Kim PS. Identification of D-peptide ligands through mirror-image phage display. Science. 1996 Mar 29;271(5257):1854-7). ; Polysomendisplay (Mattheakis LC, Bhatt RR, Dower WJ. An in vitro polysome display system for identifying ligands from very large peptide libraries. Proc Natl Acad Sci U S A. 1994 Sep 13;91(19):9022-6.), SELEX (Tuerk C. et al. RNA pseudoknots that inhibit human immunodeficiency virus type 1 reverse transcriptase.Proc Natl Acad Sci U S A. 1992 Aug 1;89(15):6988-92) oder Präsentation von Peptiden auf der Oberfläche von Bakterien (Wester-lund-Wikstrom B. Peptide display on bacterial flagella: principles and applications. Int J Med Microbiol. 2000 Jul; 290 (3):223-30.; Stahl S und Uhlen M. Bacterial surface display: trends and progress. Trends Biotechnol. 1997 May; 15(5): 185-92.)

Beispiele:

**Identifizierung von Peptiden mit spezifischen Lck-, Fyn oder Lyn-SH3-Bindungseigenschaften mittels Phagedisplay unter Einsatz eines Spezifitätswaschschrittes**

**1. Selektionen:**

**Hintergrund Phagedisplay:**

Phagendisplay ist eine Technik, die es ermöglicht Peptid-Bibliotheken mit randomisierten Aminosäuresequenzen zu konstruieren und diese nach Liganden für ein bestimmtes Zielmolekül zu durchsuchen. Die Peptidbibliothek ist dabei als Fusion aus Peptid und einem Phagenhüllprotein auf der Oberfläche von Bakteriophagen präsentiert ("phage display"). Die Diversität der präsentierten Peptide wird durch die Insertion einer kombinatorisch mutierten DNA als Peptid-kodierender Teil des Fusionsgens erreicht. Auf diese Weise wird eine extrem große Zahl von Phagen erzeugt, wobei jeder Phage ein anderes Peptid präsentiert. Das Konstruieren, Vermehren und Selektieren wird als "Biopanning" bezeichnet. Die Bibliothek wird mit einem immobilisierten Zielmolekül inkubiert, wobei der nicht-bindende Teil der Peptid-Bibliothek weggewaschen und der bindende Teil anschließend eluiert wird. Die dadurch angereicherte Population von Phagen, die ein mit dem Zielmolekül interagierendes Peptid präsentieren, werden amplifiziert, indem man mit ihnen Bakterien infiziert. Die Screening-Amplifikations-Prozedur kann mehrmals wiederholt werden, um die Bibliotheks-Mitglieder weiter anzureichern, die eine relativ höhere Affinität zum Zielmolekül besitzen. Das Ergebnis ist eine Peptid-Population, die dominiert wird von den Aminosäuresequenzen, die das Zielmolekül am besten binden.

**A. Standardprotokoll:**

Verwendet wurde eine kommerzielle Phagenbibliothek, welche die Präsentation von 12 randomisierten Aminosäuren erlaubt (Ph.D.-12™ Phage Display Library Kit, New England Biolabs, Frankfurt). Hier ist an das Phagenhüllprotein kodierende gp3-Gen nach der Signalsequenz N-terminal die Bibliothek inseriert. Diese besteht aus $1,9 \times 10^9$ unabhängigen Klonen und ist so amplifiziert und konzentriert, dass in 10 µl durchschnittlich jede Sequenz in 10 Kopien vorliegt. Zur Durchführung der Selektion gegen die SH3 Domäne der menschlichen T-Zell Tyrosinkinase (Lck SH3) wurde rekombinant hergestellte Lck SH3 als Fusion mit Glutathion-S-Transferase (GST-Lck-SH3) in einer Konzentration von 100 µg/ml in Tris-Bis-Puffer (20 mM Tris-Bis, 150 mM NaCl, pH 6,5) in einer Mikrotiterplattenvertiefung immobilisiert. Hierfür wurden jeweils 200 µl Proteinlösung über Nacht bei 4°C unter sanftem Schütteln inkubiert. Nach Entfernen der Lösung wurden verbleibende Bindungsstellen der Mikrotiterplattenvertiefung mit BSA (10 mg/ml in Tris-Bis) blockiert (60 Minuten, Inkubation unter leichtem Schütteln). Danach wurden 6 Waschschritte mit 200 µl Tris-Bis durchgeführt. Für die Selektion wurden 10 µl Phagen der kommerziellen Phagenbibliothek in 100 µl Tris-Bis mit 0,1 % Tween (Tris-BisT) für 20 Minuten unter leichtem Schütteln in einer GST-Lck-SH3-beschichteten Mikrotiterplattenvertiefung inkubiert. Die nicht gebundenen Phagen wurden verworfen und anschließend 10mal mit 200 µl Tris-BisT gewaschen. Die Elution erfolgte mit 100 µl 0,2 M Glycin-HCl, pH 2,2 für 10 Minuten unter leichtem Schütteln. Das Eluat wurde durch Zugabe von 25 µl 1 M Tris-HCl, pH 9,1 neutralisiert. Zur Amplifikation der im Eluat enthaltenen Phagen wurde 9/10 des Eluats zu 20 ml E. coli-Kultur ($OD_{600}$ von 0,1) gegeben und für 5 Stunden bei 37 °C unter starker Belüftung inkubiert. Anschließend wurde die Kultur 20 Minuten bei 5000 rpm zentrifugiert. Der Überstand wurde überführt und 1/6 Volumen PEG/NaCl (20% Polyethylenglycol-8000, 2,5 M NaCl) zugegeben. Der Ansatz wurde nun über Nacht auf Eis gefällt. Danach wurde 60 Minuten bei 5000 rpm zentrifugiert, der Überstand verworfen und das Pellet in 1 ml TBS (50 mM Tris-HCl, pH 7,5, 150 mM NaCl) resuspendiert. Anschließend wurde 5 Minuten bei 10000 rpm zentrifugiert. Der Überstand wurde überführt und 1/6 Volumen PEG/NaCl zugegeben. Der Ansatz wurde 1 Stunde auf Eis gefällt. Abschließend wurde 60 Minuten zentrifugiert und das Pellet in 100 µl TBS resuspendiert. Die so erhaltenen Phagen konnten nun in der nächsten Runde eingesetzt werden. Es wurden drei Selektionsrunden durchgeführt. Anschließend wurden willkürlich ausgewählte Phagenklone isoliert und die Aminosäuresequenz der auf diesen Phagen präsentierten Peptide über den Umweg der DNA-Sequenzierung des im Phagengenom kodierten Peptids bestimmt.

Abwandlung vom Standardprotokoll: Spezifitätswaschschritt

Anstelle des reinen Bis-Tris-Puffers wurde für die Waschschritte ein Puffer verwendet, welcher mit einer Lösung aus verschiedenen Kompetitor-SH3 Domänen (siehe Tabelle) supplementiert war, eingesetzt. In der ersten Selektionsrunde wurde mit einer Endkonzentration von 1 nM, für die Runden zwei, drei und vier, mit Endkonzentrationen von 10 nM, 100 nM und 1 µM supplementiert.

**Tabelle 1: Zusammensetzung der Kompetitorengemische, die dem Waschpuffer zugesetzt wurden**

| Köder SH3-Domäne | Zusammensetzung des SH3-Kompetitorengemisches |
|---|---|
| Fyn | Src, Hck, Lyn, Lck, Abl, Pi3k |
| Lyn | Fyn, Src, Hck, Lck, Abl, Pi3k |
| Lck | Src, Hck, Lyn, Abl, Pi3k |

**Ergebnisse:**

Gezeigt sind alle Peptid-Sequenzen, die am Ende der jeweiligen Selektion nach Sequenzierung zufällig ausgewählter Phagenklone erhalten wurden. Die Zahl in Klammern gibt an, wie oft die jeweilige Sequenz erhalten wurde. Die fett markierten Sequenzen wurden näher auf ihre Bindungseigenschaften zum Ködermolekül sowie zu anderen SH3-Domänen charakterisiert.

Standardselektion mit Lck-SH3 als Köder:

**>PD1_1(11)s entspricht Kontrollpeptid**
**HSKYPLPPLPSL**
>PD2_1(5)s
AHHHWHPLPTLP
>PD3_1(1)s
HPGYPLPPFPLP
>PD4_1(1)s
HTWHPLPILPPK

Selektion mit Spezifitätswaschschritt und Lck-SH3 als Köder:

>PD1_1(1)
HSKYPLPPLPSL
>PDII1_1(12) **entspricht** Sequenz_1
**HSWYPVPIPPSS**
**>PDII2_1(2) entspricht Sequenz_2**
**SVSPTWPLPPFP**
>PDII3_(1)
HLWHLPPRTPTA
>PDII4_(1)
APGDMLPPLPHL
>PD5_1(1)
SVSVGMKPSPRP

Selektion mit Spezifitätswaschschritt und Fyn-SH3 als Köder:

>**Fyn36_1(18)k entspricht** Sequenz_4
**HHPAQRPLPDPP**
>Fyn25(1)k
NTLAKRPLPAVP
>Fyn14(1)k
QNHYNRPLPPVP
>**Fyn29(1)k** entspricht **Sequenz_3**
**HYNQNIPLPPLP**

>Fyn34_1(2)k
TNQNRPLPPPPT
>Fyn33(1)k
PPKTDRPLPPLP
>Fyn38_1(3)k
SPSTRPLPPIPA
>Fyn31(1)k
APGNPLPHPPPA

Selektion mit Spezifitätswaschschritt und Lyn-SH3 als Köder:

>Lyn40k
HHNMHRPLPARP
**>Lyn39_(27)1k** entspricht **Sequenz_5**
HHLDNRSLPPRP
>Lyn11k
HNQLNRPLPPPP
>Lyn20_1 (3)k
QNHYNRPLPPVP
>Lyn21k
QFTARPLPPLPL
>Lyn08k
HHKISPLPAIPL
>Lyn 17k
APGNPLPHPPPA
>Lyn15k
VTMSSEASKRAP

## 2. Charakterisierung der SH3 Bindungseigenschaften selektierter Peptide

**"anti-Phage-ELISA" - Bestimmung der relativen SH3-Bindungsaktivität einzelner Phagenklone**

Dieser Bindungstest ist ein häufig verwendeter Test zur schnellen Bestimmung relativer Bindungsstärken selektierter Peptide, die hierbei noch physikalisch mit dem Phagen verknüpft vorliegen. Dieser Schritt dient auch zur Eliminierung von (bei dieser Methode nicht selten auftretenden) Phagenvarianten, die z. B. aufgrund ihrer Affinität zur Plastikoberfläche der verwendeten Mikrotiterplatten, angereichert werden und die somit zur Verfälschung der Ergebnisse führen können. Eine grundsätzliche Beschreibung der Methode findet sich z. B. unter Sparks et al. Methods Enzymol. 1995; 255:498-509.

Standardprotokoll:

GST-SH3 Protein wurde in einer Konzentration von 1-10 μg/ml in Tris-Bis über Nacht bei 4°C in Mikrotiterplatten immobilisiert. Verbleibende Bindungsstellen wurden durch 2-stündige Inkubation mit Blockierungspuffer (Bis-Tris mit 2 % Magermilchpulver) abgesättigt. In einer anderen Mikrotiterplatte wurden zuerst je 100 μl Suspension der zu testenden Phagenvariante mit 100 μl Blockierungspuffer für 20 Minuten vorinkubiert und erst dann für 1 Stunde zum immobilisierten GST-SH3 Protein gegeben. Nach einem Waschschritt mit Bis-Tris wurde ein Meerrettich-Peroxidase-konjugierter Antikörper zugegeben und die Platten für eine weitere Stunde inkubiert. Nach weiteren drei Waschschritten mit Bis-Tris konnten die gebundenen Phagen mittels einer Farbreaktion unter Verwendung von 3,3',5,5'-Tetramethylbenzidine/Wasserstoffperoxid nach Abstoppen mit 100 μl Schwefelsäure bei 450 nm detektiert werden (Brüsselbach S, Korn T, Völkel T, Müller R, Konterman RE. Enzyme recruitment and tumor cell killing in vitro by a secreted bispecific single-chain diabody. Tumor Targeting 1999; 4:115-123). Die Intensität der Gelbfärbung wurde mittels eines Fluostar Optima Mikrotiterplatten-Lesegerätes (BMG Labtechnologies GmbH, Offenburg) dokumentiert und gibt die relative Bindungsstärke der getesteten Phagenvariante an.

**Oberflächen-Plasmonen-Resonanz (Biacore)-Messungen: Quantitative Bestimmung der SH3-Bindungsaktivität einzelner Peptide am Beispiel der Peptidsequenz 1**

Zur Messung der Ligand(SH3)-Analyt(Peptid)-Interaktionen wurde der CM5 Sensorchip (Biacore AB, Uppsala) benutzt. Es wurde eine Amin-Kopplung der verschiedenen SH3-Domänen (Lck, Hck, Lyn, Src) an die Dextran-Matrix durchgeführt.

| Laufpuffer (HBS) | Kopplungspuffer (Natriumacetat) |
| --- | --- |
| 0.01M HEPES | siehe Herstellerangaben ; der pH-Wert des |
| 0.15M NaCl | Puffers richtet sich nach dem isoelektrischen |
| 3mM EDTA | Punkt des Biacore-Liganden (in diesem Fall |
| 0.005% Surfactant P20 | der Lck-SH3-Domäne) |
| pH 7.4 | |

Sensorgramme wurden mit dem auf SPR ("surface plasmon resonance") basierenden Biosensor Biacore X und der Software BIACORE X Control Software (Biacore AB, Uppsala) aufgenommen. Messungen wurden mit einer Datenerfassungsgeschwindigkeit von 1 oder 5 Hz, bei einer Temperatur von 21,5°C durchgeführt. Die Flussgeschwindigkeit betrug 5 $\mu$l/min und es wurden zur Aktivierung 35 $\mu$l eines NHS / EDC-Gemisches injiziert (0,05 M NHS, 0,2 M EDC).
In der Kopplungsphase wurden positiv geladene SH3-Domänen-Lösungen in Kopplungspuffer mit Konzentrationen zwischen 0,2 und 200 $\mu$M injiziert. Dabei wurden auch Änderungen der Injektionsvolumina (20-35 $\mu$l) und Repetitionen durchgeführt.
Nach Zugabe der einzelnen SH3-Domänen wurde der Sensorchip mit 35 $\mu$l Ethanolamin (1 M) deaktiviert.
Diese Prozedur wurde für beide Flusszellen (Fc) getrennt durchgeführt, dabei wurde an Fc2 die SH3-Domäne gekoppelt und anschließend Fc1 mit der gleichen Vorgehensweise ohne SH3-Protein behandelt. Um nicht kovalent gebundenes SH3-Protein vom Chip zu waschen, wurden 5 $\mu$l Guanidinhydrochlorid (6 M) injiziert.
Mit Kopplungen und Bindungsstudien konnte erst nach Äquilibrierung des BiacoreX Systems begonnen werden, also nach Erreichen einer stabilen Basislinie. Die eingesetzte Flussgeschwindigkeit für Konzentrationsreihen wurde von 5-25 $\mu$l/min variiert. Für die einzelnen Sensorgramme wurden Peptidlösungen (PDII1; Sequenz 1) im Laufpuffer zwischen 0,2 und 300 $\mu$M hergestellt. Nach Erreichen der Basislinie wurden die Peptidproben injiziert. Die Konzentrationsreihe wurde so lange fortgesetzt bis sich keine nennenswerten Höhenveränderungen am Plateau der Injektionssensorgramme zeigten.

Die Prozessierung, der mit der BIACORE X Control Software gewonnen Daten wurde mit der Biaevaluation Software (Biacore AB, Uppsala) durchgeführt. Die Daten der Fc2 wurden mit den entsprechenden Daten der Fc1 referenziert, dabei wird das Sensorgramm einer Flusszelle ohne Ligand (SH3-Protein) von der Flusszelle mit Liganden subtrahiert. Alle Injektionen aus einem Versuch wurden in einem Plot übereinander gelegt und in dieser Form ausgewertet. Die Dissoziationskonstante $K_D$ wurde aus dem Gleichgewichtszustand ermittelt. Es wurde das einfachste 1:1 Bindungsmodel als Grundlage für die Auswertung benutzt.
Der Fit erfolgte mit der Biaevaluation Software nach folgender Formel:

$$R = \frac{R_{eq} \cdot c}{K_D + c}$$

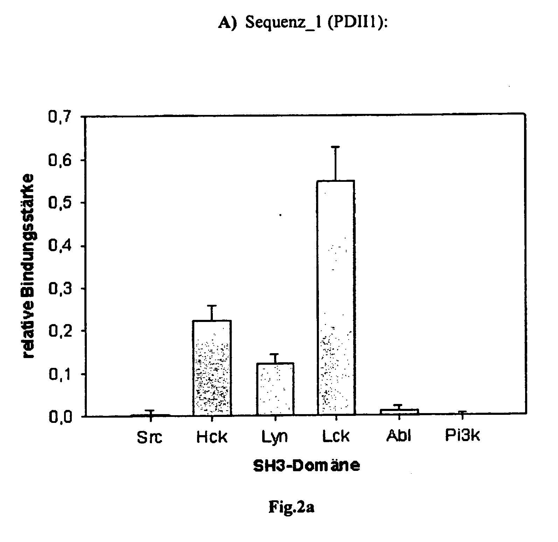

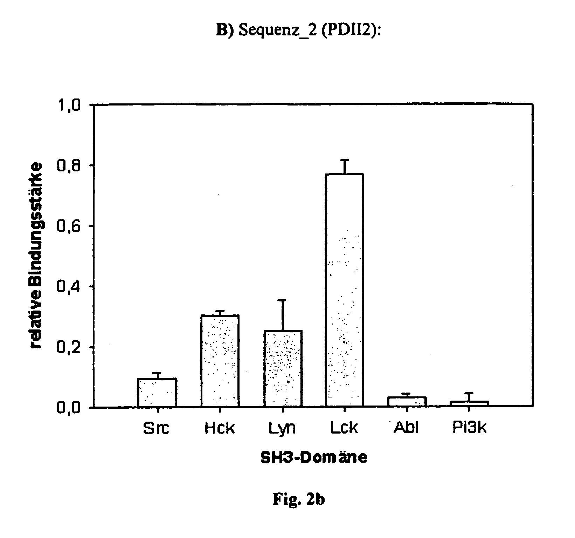

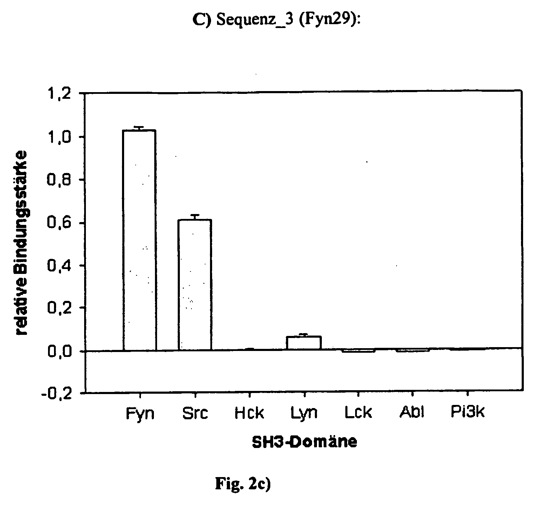

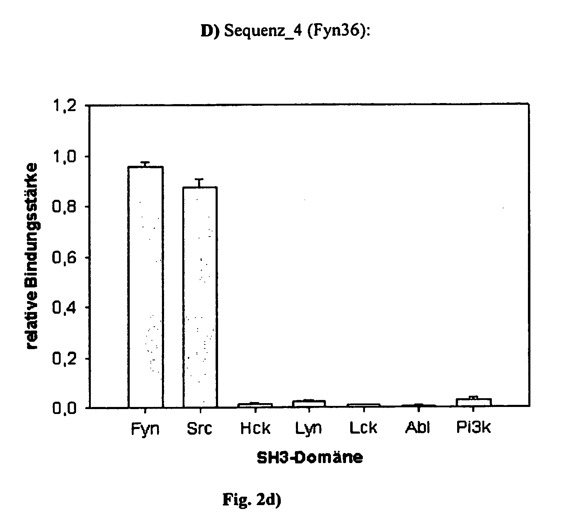

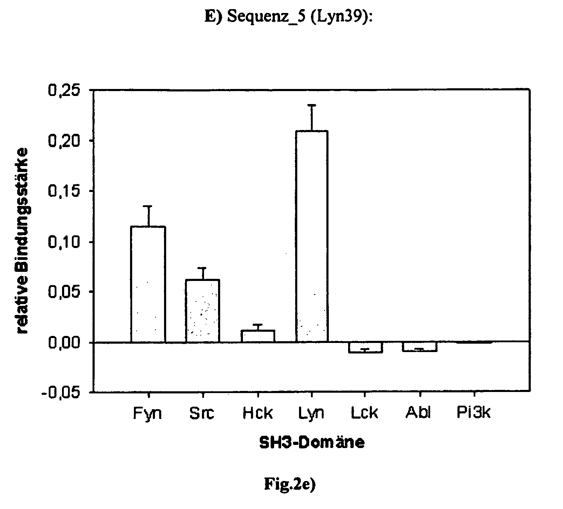

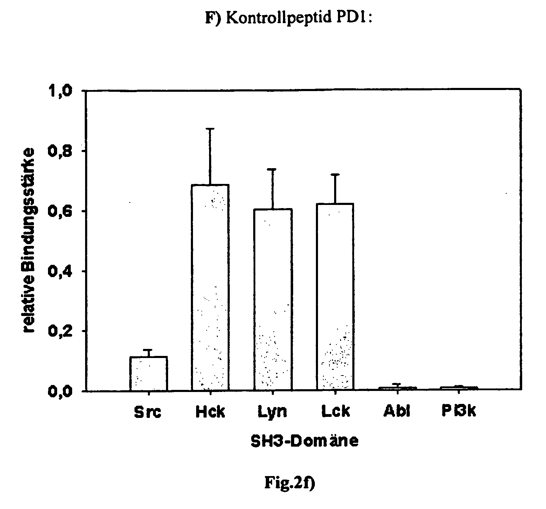

Die Ergebnisse der Messung der relativen Bindungsaktivitäten mittels "anti-Phagen-ELISA" sind in den Figuren 2a) bis 2f) dargestellt, die Ergebnisse der Biacore-Messungen für das Peptid PDII1 (Sequenz 1) sind in nachfolgender Tabelle 2 dargestellt.

Tabelle 2: Quantitative SH3-Bindungsaktivität des spezifischen Peptides PDII1 (Sequenz 1). Die Dissoziationskonstante wurde mittels Oberflächen-Plasmonen-Resonanz-Messung ermittelt.

| SH3-Domäne | $K_D$-Wert [$\mu$M] |
| --- | --- |
| Lck | 6 |
| Hck | 10 |
| Lyn | 27 |

(fortgesetzt)

| SH3-Domäne | $K_D$-Wert [µM] |
|---|---|
| Src | >200 |

Die Ergebnisse des Antiphagen-Elisas sind in den Figuren 2a) bis 2f) dargestellt.

Fig. 2a)-f) zeigt die Bestimmung der relativen SH3-Bindungsaktivität der beiden spezifisch an die Lck-SH3 bindenden Peptide (Sequenzen 1 und 2; A und B), der beiden spezifisch an die Fyn-SH3 bindenden Peptide (Sequenzen 3 und 4; C und D) sowie des spezifisch an die Lyn-SH3 bindenden Peptides (5, E) und eines Kontrollpeptides PD1 (F) mittels "anti-Phage"-ELISA.

Es wurde die relative Bindungsaktivität der Phagenvarianten, welche die entsprechenden Peptidsequenzen präsentieren, an verschiedene SH3-Domänen bestimmt. Unter den Balken sind die Namen der jeweiligen SH3-Domäne angegeben, welche für den Bindungstest eingesetzt wurde. Diejenige SH3-Domäne, gegen welche die jeweilige Sequenz selektiert worden war, ist in A und B Lck-SH3, in C und D Fyn-SH3 und in E Lyn-SH3. Alle Sequenzen zeigen bei derjenigen SH3-Domäne, die während der Selektion als Köder eingesetzt wurde, die stärkste Bindung und besitzen somit spezifische Bindungseigenschaften. Diese Ergebnisse können nur erreicht werden, wenn während der Selektion ein Spezifitätswaschschritt unter Einsatz von Kompetitoren erfolgt. Wäscht man nur mit Puffer, erhält man zwar auch an den jeweiligen Köder bindende Moleküle, diese binden aber auch an zum Köder ähnliche Moleküle. Peptid "PD1" stammt aus einer Standardselektion mit Lck-SH3 als Köder, man erkennt, dass dieses Peptid zur Lck-SH3 nicht die stärkste Bindung aufweist, sondern ähnlich gut an Hck- und Lyn-SH3 binden kann.

**Patentansprüche**

1. Protein nach Sequenz 2.

2. Therapeutisches Mittel,
   **dadurch gekennzeichnet,**
   **dass** es eine Struktur nach der Sequenz 2 besitzt.

3. Mittel zur Behandlung der Krankheit Krebs und zur Senkung der Pathogenität der Viren HIV-1 (Nef-Protein) und Hepatitis C (NS5A-Protein),
   **dadurch gekennzeichnet,**
   **dass** es eine Struktur nach der Sequenz 2 besitzt.

**Claims**

1. Protein according to Sequence 2.

2. Therapeutic agent, **characterised in that** said agent has a structure according to Sequence 2.

3. Agent for treating the disease cancer and for reducing the pathogenicity of the viruses HIV-1 (Nef protein) and hepatitis C (NSSA protein), **characterised in that** said agent has a structure according to Sequence 2.

**Revendications**

1. Protéine selon la séquence 2.

2. Moyen thérapeutique,
   **caractérisé en ce**
   **qu'**il possède une structure selon la séquence 2.

3. Moyen pour le traitement d'un cancer et pour la réduction de la pathogénicité des virus du VIH-1 (protéine Nef) et de l'hépatite C (protéine NS5A),
**caractérisé en ce**
**qu'**il possède une structure selon la séquence 2.

Fig.1

**A)** Sequenz_1 (PDII):

**Fig.2a**

**B)** Sequenz_2 (PDII2):

**Fig. 2b**

C) Sequenz_3 (Fyn29):

Fig. 2c)

**D)** Sequenz_4 (Fyn36):

**Fig. 2d)**

E) Sequenz_5 (Lyn39):

Fig.2e)

**F) Kontrollpeptid PD1:**

Fig.2f)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **PARMLEY SF ; SMITH GP.** Antibody-selectable filamentous fd phage vectors: affinity purification of target genes. *Gene,* 20. Dezember 1988, vol. 73 (2), 305-18
- Beyer Walter. 829-834
- **STRAUS DB ; WEISS A.** Genetic evidence for the involvement of the lck tyrosine kinase in signal transduction through the T cell antigen receptor. *Cell,* 21. August 1992, vol. 70 (4), 585-93
- **LAWRENCE DS ; NIU J.** Protein kinase inhibitors: the tyrosine-specific protein kinases. *Pharmacol Ther.,* Februar 1998, vol. 77 (2), 81-114
- **RESH MD.** Fyn, a Src family tyrosine kinase. *Int J Biochem Cell Biol.,* November 1998, vol. 30 (11), 1159-62
- **HUANG J et al.** Cooperative roles of Fyn and cortactin in cell migration of metastatic murine melanoma. *J Biol Chem.,* 28. November 2003, vol. 278 (48), 48367-76
- **BERWANGER B et al.** Loss of a FYN-regulated differentiation and growth arrest pathway in advanced stage neuroblastoma. *Cancer Cell,* November 2002, vol. 2 (5), 377-86
- **XU Y et al.** Lyn tyrosine kinase: accentuating the positive and the negative. *Immunity,* Januar 2005, vol. 22 (1), 9-18
- **STETTNER MR.** Lyn kinase activity is the predominant cellular SRC kinase activity in glioblastoma tumor cells. *Cancer Res.,* 01. Juli 2005, vol. 65 (13), 5535-43

- **CONTRI A et al.** Chronic lymphocytic leukemia B cellscontain anomalous Lyn tyrosine kinase, a putative contribution to defective apoptosis. *J Clin Invest.,* Februar 2005, vol. 115 (2), 369-78
- **SCHUMACHER TN ; MAYR LM ; MINOR DL JR ; MILHOLLEN MA ; BURGESS MW ; KIM PS.** Identification of D-peptide ligands through mirror-image phage display. *Science,* 29. Marz 1996, vol. 271 (5257), 1854-7
- **MATTHEAKIS LC ; BHATT RR ; DOWER WJ.** An in vitro polysome display system for identifying ligands from very large peptide libraries. *Proc Natl Acad Sci U S A.,* 13. September 1994, vol. 91 (19), 9022-6
- **TUERK C. et al.** RNA pseudoknots that inhibit human immunodeficiency virus type 1 reverse transcriptase. *Proc Natl Acad Sci U S A.,* 01. August 1992, vol. 89 (15), 6988-92
- **WESTER-LUND-WIKSTROM B.** Peptide display on bacterial flagella: principles and applications. *Int J Med Microbiol.,* Juli 2000, vol. 290 (3), 223-30
- **STAHL S ; UHLEN M.** Bacterial surface display: trends and progress. *Trends Biotechnol.,* Mai 1997, vol. 15 (5), 185-92
- **SPARKS et al.** *Methods Enzymol.,* 1995, vol. 255, 498-509
- **BRÜSSELBACH S ; KORN T ; VÖLKEL T ; MÜLLER R ; KONTERMAN RE.** Enzyme recruitment and tumor cell killing in vitro by a secreted bispecific single-chain diabody. *Tumor Targeting,* 1999, vol. 4, 115-123

SEQUENCE LISTING

    <110> Forschungszentrum Jülich GmbH

    <120> Spezifisch an einen Köder bindende Biomoleküle

    <130> PT1.2286

    <160> 26

    <170> PatentIn version 3.5

    <210> 1
    <211> 12
    <212> PRT
    <213> Artificial

    <220>
    <223> Selektiert mit Lck-SH3 als Köder mit Spezifitätswaschschritt

    <400> 1

```
            His Ser Trp Tyr Pro Val Pro Ile Pro Pro Ser Ser
            1               5                   10
```

    <210> 2
    <211> 12
    <212> PRT
    <213> Artificial

    <220>
    <223> Selektiert mit Lck-SH3 als Köder mit Spezifitätswaschschritt

    <400> 2

```
Ser Val Ser Pro Thr Trp Pro Leu Pro Pro Phe Pro
1               5               10
```

<210> 3
<211> 12
<212> PRT
<213> Artificial

<220>
<223> Selektiert mit Spezifitätswaschschritt und Fyn-SH3 als Köder

<400> 3

```
His Tyr Asn Gln Asn Ile Pro Leu Pro Pro Leu Pro
1               5               10
```

<210> 4
<211> 12
<212> PRT
<213> Artificial

<220>
<223> Selektiert mit Spezifitätswaschschritt und Fyn-SH3 als Köder

<400> 4

```
His His Pro Ala Gln Arg Pro Leu Pro Asp Pro Pro

    1               5                   10
```

<210> 5
<211> 12
<212> PRT
<213> Artificial

<220>
<223> Selektiert mit Spezifitätswaschschritt und Lyn-SH3 als Köder

<400> 5

```
His His Leu Asp Asn Arg Ser Leu Pro Pro Arg Pro
1               5               10
```

<210> 6
<211> 12
<212> PRT
<213> Artificial

<220>
<223> Kontrollpeptid

<400> 6

```
His Ser Lys Tyr Pro Leu Pro Pro Leu Pro Ser Leu
1               5               10
```

<210> 7

<210> ... (continued)

<211> 12
<212> PRT
<213> Artificial

<220>
<223> Selektion mit Lck-SH3 als Köder

<400> 7

```
Ala His His His Trp His Pro Leu Pro Thr Leu Pro
1               5                   10
```

<210> 8
<211> 13
<212> PRT
<213> Artificial

<220>
<223> Selektiert mit Lck-SH3 als Köder

<400> 8

```
Leu His Pro Gly Tyr Pro Leu Pro Pro Phe Pro Leu Pro
1               5                   10
```

<210> 9
<211> 12
<212> PRT
<213> Artificial

<220>
<223> Selektiert mit Lck-SH3 als Köder

<400> 9

```
His Thr Trp His Pro Leu Pro Ile Leu Pro Pro Lys
1               5                   10
```

<210> 10
<211> 12
<212> PRT
<213> Artificial

<220>
<223> Selektiert mit Lck-SH3 als Köder mit Spezifitätswaschschritt

<400> 10

```
His Ser Lys Tyr Pro Leu Pro Pro Leu Pro Ser Leu
1               5                   10
```

<210> 11
<211> 12
<212> PRT
<213> Artificial

<220>
<223> Selektiert mit Lck-SH3 als Köder mit Spezifitätswaschschritt

<400> 11

```
                    His Leu Trp His Leu Pro Pro Arg Thr Pro Thr Ala
                    1               5               10
```

<210> 12
<211> 12
<212> PRT
<213> Artificial

<220>
<223> Selektiert mit Lck-SH3 als Köder mit Spezifitätswaschschritt

<400> 12

```
                    Ala Pro Gly Asp Met Leu Pro Pro Leu Pro His Leu
                    1               5               10
```

<210> 13
<211> 12
<212> PRT
<213> Artificial

<220>
<223> Selektiert mit Lck-SH3 als Köder mit Spezifitätswaschschritt

<400> 13

```
                    Ser Val Ser Val Gly Met Lys Pro Ser Pro Arg Pro
                    1               5               10
```

<210> 14
<211> 12
<212> PRT
<213> Artificial

<220>
<223> Selektiert mit Spezifitätswaschschritt und Fyn-SH3 als Köder

<400> 14

```
                    Asn Thr Leu Ala Lys Arg Pro Leu Pro Ala Val Pro
                    1               5               10
```

<210> 15
<211> 12
<212> PRT
<213> Artificial

<220>
<223> Selektiert mit Spezifitätswaschschritt und Fyn-SH3 als Köder

<400> 15

```
                    Gln Asn His Tyr Asn Arg Pro Leu Pro Pro Val Pro
                    1               5               10
```

<210> 16

<211> 12
<212> PRT
<213> Artificial

<220>
<223> Selektiert mit Spezifitätswasschritt und Fyn-SH3 als Köder

<400> 16

```
Thr Asn Gln Asn Arg Pro Leu Pro Pro Pro Pro Thr
1               5               10
```

<210> 17
<211> 12
<212> PRT
<213> Artificial

<220>
<223> Selektiert mit Spezifitätswaschschritt und Fyn-SH3 als Köder

<400> 17

```
Pro Pro Lys Thr Asp Arg Pro Leu Pro Pro Leu Pro
1               5               10
```

<210> 18
<211> 12
<212> PRT
<213> Artificial

<220>
<223> Selektiert mit Spezifitätswaschschritt und Fyn-SH3 als Köder

<400> 18

```
Ser Pro Ser Thr Arg Pro Leu Pro Pro Ile Pro Ala
1               5               10
```

<210> 19
<211> 12
<212> PRT
<213> Artificial

<220>
<223> Selektiert mit Spezifitätswaschschritt und Fyn-SH3 als Köder

<400> 19

```
Ala Pro Gly Asn Pro Leu Pro His Pro Pro Pro Ala
1               5               10
```

<210> 20
<211> 12
<212> PRT
<213> Artificial

<220>
<223> Selektiert mit Spezifitätswaschschritt und Lyn-SH3 als Köder

<400> 20

```
                    His His Asn Met His Arg Pro Leu Pro Ala Arg Pro
                    1               5               10
```

<210> 21
<211> 12
<212> PRT
<213> Artificial

<220>
<223> Selektiert mit Spezifitätswaschschritt und Lyn-SH3 als Köder

<400> 21

```
                    His Asn Gln Leu Asn Arg Pro Leu Pro Pro Pro Pro
                    1               5               10
```

<210> 22
<211> 12
<212> PRT
<213> Artificial

<220>
<223> Selektiert mit Spezifitätswaschschritt und Lyn-SH3 als Köder

<400> 22

```
                    Gln Asn His Tyr Asn Arg Pro Leu Pro Pro Val Pro
                    1               5               10
```

<210> 23
<211> 12
<212> PRT
<213> Artificial

<220>
<223> Selektiert mit Spezifitätswaschschritt und Lyn-SH3 als Köder

<400> 23

```
                    Gln Phe Thr Ala Arg Pro Leu Pro Pro Leu Pro Leu
                    1               5               10
```

<210> 24
<211> 12
<212> PRT
<213> Artificial

<220>
<223> Selektiert mit Spezifitätswaschschritt und Lyn-SH3 als Köder

<400> 24

```
                    His His Lys Ile Ser Pro Leu Pro Ala Ile Pro Leu
                    1               5               10
```

<210> 25

<211> 12
<212> PRT
<213> Artificial

<220>
<223> Selektiert mit Spezifitätswaschschritt und Lyn-SH3 als Köder

<400> 25

```
              Ala Pro Gly Asn Pro Leu Pro His Pro Pro Pro Ala
              1               5                   10
```

<210> 26
<211> 12
<212> PRT
<213> Artificial

<220>
<223> Selektiert mit Spezifitätswaschschritt und Lyn-SH3 als Köder

<400> 26

```
              Val Thr Met Ser Ser Glu Ala Ser Lys Arg Ala Pro
              1               5                   10
```